# FASCICULE DE BREVET EUROPEEN

(11) **EP 1 155 685 B1**
(45) Date de publication et mention de la délivrance du brevet: **09.02.2005**
(21) Numéro de dépôt: 01401109.2
(22) Date de dépôt: 27.04.2001
(51) Int. Cl.: A61K 7/48

(54) **Composition comprenant du manganèse**
Zusammensetzung enthaltend Mangan
Composition containing manganese

(30) Priorité: 18.05.2000 FR 0006374
(43) Date de publication de la demande: 21.11.2001
(73) Titulaire: L'OREAL, 75008 Paris (FR)
(72) Inventeur: Nonotte, Isabelle, 75017 Paris (FR); Breton, Lionel, 78000 Versailles (FR)
(74) Mandataire: Galup, Cédric Olivier Nicolas

(56) Documents cités:
- EP-A- 0 424 033
- EP-A- 0 770 392
- WO-A-96/19182

## Description

La présente invention se rapporte à l'utilisation cosmétique d'une quantité efficace du manganèse ou de ses sels, dans une composition cosmétique, pour combattre combattre la pâleur de la peau. Elle se rapporte également à l'utilisation d'une quantité efficace du manganèse pour la préparation d'une composition destinée à rétablir l'équilibre vasculaire de la peau modifié après un stress.

Le stress physiologique correspond à tout changement externe ou interne auquel l'organisme doit s'adapter pour maintenir sa santé et/ou sa survie. Il met en jeu différent processus hormonaux chronologiques. En effet, en réaction à toutes agressions externes ou internes, le système nerveux central envoie des messages hormonaux via la circulation sanguine aux glandes surrénaliennes en particulier la glande corticosurrénalienne. Cette dernière libère le cortisol qui va agir directement sur la glande médulosurrénalienne voisine où il stimulera la libération d'adrénaline. En réponse, l'adrénaline véhiculée par la circulation sanguine agit sur les principaux organes (coeur, foie, ...) et les grandes fonctions physiologiques afin de préserver l'organisme et de maintenir son homéostasie. En parallèle, le système nerveux sympathique va agir sur la circulation sanguine périphérique notamment au niveau cutané grâce à la libération de noradrénaline au niveau des terminaisons nerveuses contrôlant avec les microvaisseaux du derme superficiel.

La noradrénaline est stockée dans des vésicules granuleuses le long des terminaisons nerveuses postganglionnaires sympathiques. Sa libération survient lorsqu'un potentiel d'action atteint la synapse en contact avec le muscle lisse des microvaisseaux de la circulation sanguine cutanée. L'arrivée de ce potentiel d'action entraîne l'ouverture de canaux calciques au niveau de la membrane cellulaire et donc l'apparition d'un courant de Ca²⁺ conduisant à l'exocytose des vésicules noradrénergiques et à la libération de noradrénaline dans la fente de la jonction nerf/muscle.
La noradrénaline ainsi libérée se fixe sur les récepteurs de type • présents sur la membrane plasmique des cellules musculaires lisses. La cascade biochimique intracellulaire activée par cette fixation, fait intervenir les seconds messagers tels que l'inositol triphosphate et le calcium intracellulaire. A l'état basal, la concentration en ions Ca²⁺ dans le cytoplasme est très faible (environ 0.1µM) et est maintenue en permanence à ce niveau grâce à des pompes ATP dépendantes: L'activation des récepteurs de type • déclenche une augmentation soudaine du niveau de Ca²⁺ cytoplasmique grâce à l'ouverture de canaux calciques présents dans la membrane plasmique ou dans la membrane entourant les stocks de calcium intracellulaire contenus dans le réticulum endoplasmique. Le Ca²⁺ ainsi libéré et activant des mécanismes intracellulaires, conduit à une vasoconstriction du muscle lisse des microvaisseaux cutanés.

Au cours du stress, des quantités importantes de noradrénaline sont donc libérées par le système nerveux périphérique. Parallèlement, la circulation sanguine véhicule des doses massives d'adrénaline directement libérée des glandes surrénales. L'ensemble des catécholamines ainsi apporté dans le compartiment cutané, génère différentes modifications et notamment une vasoconstriction des microvaisseaux du derme qui se traduit par un aspect du visage particulier. C'est la pâleur associée au stress.

On comprend donc de ce qui précède que la pâleur que l'on peut associer au stress est en partie induite par une variation de flux de calcium au travers des canaux calciques transmembranaires des microvaisseaux cutanés

Personne n'avait, jusqu'à ce jour, établi un lien entre les canaux calcium du tissu vasculaire sous cutanés, le manganèse et la pâleur consécutive à un stress, et n'avait trouvé qu'il était possible de traiter ces phénomènes en agissant sur les canaux calcium notamment via le manganèse.

Ainsi, la demanderesse propose d'agir sur les canaux calciques pour relâcher ou relaxer les tissus vasculaires cutanés, et ainsi combattre la pâleur de la peau

Le manganèse est un métal très répandu à la surface de la croûte terrestre.
Il appartient au groupe Vlla de Mendeleïev, son numéro atomique est 25, son poids atomique 54,93. Le manganèse présente plusieurs valences (1 à 7), les formes di- et trivalentes sont celles qui sont biologiquement les plus actives.

Le manganèse est très utilisé dans l'industrie métallurgique, dans la fabrication de piles sèches et comme colorant.

Les végétaux sont tous riches en Mn : particulièrement les graines (environ 7 µg/g), les noix (environ 17 µg/g) et le thé. Les fruits (environ 1 µg/g) et les légumes (environ 2,5 µg/g) sont moins riches, mais leur taux est encore très élevé par rapport aux aliments d'origine animale (viandes : environ 0,20 µg/g, poissons : environ 0,05 µg/g).
A l'inverse chez les animaux, particulièrement l'homme, ce métal ne se trouve qu'à l'état de trace.

Son rôle biologique est cependant très important et même, si les effets néfastes d'une carence n'ont pas été constatés de façon irréfutable chez l'Homme, les conséquences des déficits examinées chez l'animal montrent que ce métal est impliqué dans de nombreux métabolismes. Mais, encore aujourd'hui, les connaissances sur les mécanismes biochimiques intimes de ce métal restent très fragmentaires.

Le manganèse a été impliqué dans de nombreux métabolismes
- la coagulation ;
- la thermogenèse (par son action sur le système thyroïdien)
- l'immunité, où le manganèse semble être nécessaire à une synthèse correcte des anticorps ;
- la reproduction, sa carence entraînant une baisse de fertilité des femelles, et des mâles, peut-être à cause de l'action limitante du manganèse sur la synthèse du cholestérol et de celle des précurseurs des hormones sexuelles.

Deux propriétés permettent d'expliquer une partie du rôle physiopathologique du manganèse :
- l'activation de nombreuses enzymes.
   Le manganèse est un métal qui active de nombreuses enzymes et lectines. Il intervient soit comme un élément dissociable, soit en faisant partie intégrante de la structure de l'enzyme (métalloenzymes).
- son activité inhibitrice vis-à-vis des canaux calcium.
   Le signal interne d'activation d'une cellule est souvent déclenché par une modification des concentrations intracytoplasmiques du calcium. Ce dernier se fixe sur des protéines (calmoduline), qui vont à leur tour activer des kinases.
   Le calcium sert à la transmission de l'influx nerveux, à la stimulation de certaines cellules sécrétantes, il déclenche les changements de forme de la plaquette au début de son activation, etc.

Le manganèse bloque la pénétration du calcium vers le cytoplasme dans de nombreuses cellules à activité sécrétrice (pancréas par exemple), ou électrique ; il inhibe notamment la sortie des neurotransmetteurs au niveau de la plaque motrice. Le manganèse a une action inhibitrice sur la stimulation des lymphocytes B et T, s'il est ajouté au milieu, très peu de temps après le mitogène.

Pour qu'une substance soit reconnue comme un inhibiteur des canaux calcium, autrement appelé dans le texte antagoniste calcique, elle doit pouvoir diminuer la concentration intracellulaire en calcium ou diminuer la liaison du calcium aux protéines intracellulaires comme par exemple la calmoduline, tel que cela est notamment décrit par exemple, par Galizzi, J.P et al, J. Biol. Chem. 1987, 262 p 6947 ou Y. Okamiya et al, Eur. J. Pharmacol. 1991, 205, p 49 ou J.A. Wagner et al, J. Neurosci. 1988, 8, p 3354 ou H.R Lee et al, Life Sci. 1984, 35 p 721 ou Schoemaker H. et Lauger S. Eur. J. Pharmacol. 1985, 111 p 273 ou encore I.J. Reynolds et al, J. Pharmacol. Exp. Ther. 1986, 237 p 731.

Une substance est reconnue comme relaxante au sens de l'invention lorsqu'elle montre un effet de relaxation sur un tissu musculaire contracté et/ou montrer un effet inhibiteur dans un modèle expérimental de jonction nerf-muscle (plaque motrice) notamment dans le modèle décrit par W. Steinbrecher dans : Electrodes for stimulation and bioelectric potentiel recording, Ed. Biomerstechnich, 1988, pages 96-98.

Le manganèse ou ses sels répondent bien à ces définitions.

Comme précédemment indiqué, la demanderesse propose d'agir sur les canaux calciques pour relâcher ou relaxer les tissus vasculaires, et ainsi combattre la pâleur de la peau Pour cela, elle propose l'utilisation du manganèse qu'il soit sous la forme ionique ou sous la forme de sel ou sous la forme d'extraits naturels, végétaux ou de micro-organismes, particulièrement bactériens, riches en manganèse.

Ainsi, la présente invention se rapporte à l'utilisation cosmétique, dans une composition cosmétique dans un milieu physiologiquement acceptable, d'une quantité efficace du manganèse ou d'au moins l'un de ses sels, pour relaxer et/ou relâcher le tissu vasculaire cutané et/ou sous-cutané et ainsi combattre la pâleur de la peau.

L'invention a plus particulièrement pour objet l'utilisation d'une quantité efficace de manganèse pour la préparation d'une composition destinée à rétablir l'équilibre vasculaire de la peau modifié après un stress.

L'invention se rapporte également à l'utilisation dans une composition, dans un milieu physiologiquement acceptable, d'une quantité efficace d'extraits naturels, végétaux ou de micro-organismes, particulièrement bactériens, riches en manganèse ou en sel de manganèse, l'extrait étant destiné à relaxer et/ou relâcher le tissu vasculaire cutané et/ou sous-cutané, et ainsi combattre la pâleur de la peau. L'invention se rapporte également à l'utilisation d'une quantité efficace de manganèse pour la préparation d'une composition destinée à rétablir l'équilibre vasculaire de la peau modifié après un stress.

Par sels de manganèse on entend selon l'invention les sels organiques ou inorganiques du manganèse.

Comme sels organiques utilisables selon l'invention, on peut citer le gluconate de manganèse ou le carbonate de manganèse ou l'acétate de manganèse ou le citrate de manganèse ou l'oléate de manganèse ou l'oxalate de manganèse.

Comme sels inorganiques de manganèse on peut citer les sels minéraux comme le chlorure de manganèse ou le borate de manganèse ou le nitrate de manganèse ou le phosphate de manganèse ou le sulfate de manganèse.

Par ailleurs dans le texte, sous réserve d'indication contraire, l'emploi du terme manganèse doit être compris comme signifiant aussi bien le manganèse sous forme ionique, sous forme de sels ou sous forme d'extraits naturels, végétaux ou de micro-organismes, particulièrement bactériens, riches en manganèse.

Par milieu physiologiquement acceptable, on entend compatible avec la peau, le cuir chevelu et/ou les muqueuses.

Plus particulièrement la relaxation et/ou le relâchement du tissu vasculaire cutané et/ou sous-cutané correspond à un relâchement ou une relaxation des muscles lisses vasculaires.

La quantité efficace de manganèse utilisable selon l'invention est bien entendu fonction de l'effet recherché et peut donc varier dans une large mesure.

Pour donner un ordre de grandeur, il est possible d'utiliser selon l'invention du manganèse en une quantité représentant de 0,0001% à 10 % du poids total de la composition et préférentiellement en une quantité représentant de 0,001 % à 5 % du poids total de la composition.

Bien entendu lorsque selon l'invention on utilise un extrait naturel, végétal ou de micro-organismes, particulièrement bactériens, riche en manganèse, l'homme métier sait adapter la quantité d'extrait à utiliser pour, au final, utiliser le manganèse dans les quantités ci-dessus exposées.

Comme extraits naturels riche en manganèse utilisables selon l'invention, on peut citer les extraits de noix ou les extraits de thé.

Les compositions de l'invention sont destinées à des applications cosmétiques ou dermatologiques. Préférentiellement, les compositions de l'invention sont destinées à des applications cosmétiques.

Les utilisations selon l'invention sont cosmétiques car elles visent à modifier l'aspect de la personne.

Les compositions selon l'invention peuvent se présenter sous toutes les formes galéniques normalement utilisées pour une application topique, injectable, ou encore par voie orale.

La composition selon l'invention peut être appliquée soit par voie locale, c'est-à-dire par voie topique, ou par injection sous-cutanée et/ou intradermique.

Préférentiellement selon l'invention, la composition est appliquée par voie topique.

Les quantités des différents constituants des compositions selon l'invention sont celles classiquement utilisées dans les domaines considérés et sont appropriées à leur forme galénique.
Pour une application topique, les compositions de l'invention comprennent un milieu compatible avec la peau. Ces compositions peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Ces compositions à application topique peuvent constituer notamment une composition de protection, de soin pour le visage, pour le cou, pour les mains ou pour le corps, (par exemple crèmes de jour, crèmes de nuit, crèmes ou huiles solaires, laits corporels), une composition de maquillage (par exemple fond de teint) ou une composition de bronzage artificiel.

Quand la composition de l'invention est une émulsion, la proportion de corps gras qu'elle contient peut aller de 5 % à 80 % en poids, et de préférence de 5 % à 50 % en poids par rapport au poids total de la composition. Les corps gras et les émulsionnants utilisés dans la composition sous forme d'émulsion sont choisis parmi ceux classiquement utilisés dans le domaine cosmétique ou pharmaceutique.

Comme corps gras utilisables dans l'invention, on peut citer les huiles minérales (vaseline), les huiles végétales (fraction liquide de beurre de karité) et leurs dérivés hydrogénés, les huiles animales, les huiles de synthèse (perhydrosqualène), les huiles siliconées (diméthylpolysiloxane) et les huiles fluorées. Comme autres corps gras, on peut encore citer les alcools gras (alcool cétylique, alcool stéarylique), les acides gras (acide stéarique) et les cires.

Les émulsionnants peuvent être présents, dans la composition, en une proportion allant de 0,3 % à 30 % en poids, et de préférence de 0,5 à 30 % en poids par rapport au poids total de la composition.

De façon connue, la composition de l'invention peut contenir également des adjuvants habituels dans les domaines correspondants, tels que les gélifiants hydrophiles ou lipophiles, les conservateurs, les antioxydants, les solvants, les parfums, les charges, les filtres et les matières colorantes. Par ailleurs, ces compositions peuvent contenir des actifs hydrophiles ou lipophiles. Les quantités de ces différents adjuvants ou actifs sont celles classiquement utilisées dans le domaine cosmétique ou pharmaceutique, et par exemple de 0,01 % à 20 % du poids total de la composition. Ces adjuvants ou ces actifs, selon leur nature, peuvent être introduits dans la phase grasse, dans la phase aqueuse et/ou dans des vésicules lipidiques.

Parmi les actifs que peuvent contenir les compositions de l'invention, on peut notamment citer les actifs ayant un effet sur le traitement des rides ou des ridules, autres que le manganèse, et en particulier les actifs kératolytiques. Par kératolytique, on entend un actif ayant des propriétés desquamantes, exfoliantes ou gommantes, ou un actif capable de ramollir la couche cornée.

Parmi les actifs ayant un effet sur le traitement des rides ou des ridules que peuvent contenir les compositions de l'invention, on peut citer par exemple l'alvérine, les ouvreurs de canaux chlore, les hydroxyacides et les rétinoïdes.

Les hydroxyacides peuvent être par exemple des α-hydroxy-acides ou des β-hydroxy-acides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Les hydroxyacides qui peuvent être utilisés sont notamment les acides glycolique, lactique, malique, tartrique, citrique, hydroxy-2 alcanoïque, mandélique, salicylique, ainsi que leurs dérivés alkylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthyl-benzoïque, ou encore leurs dérivés alcoxylés comme l'acide 2-hydroxy-3-méthoxybenzoïque.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol, ainsi que leurs sels.

Ces actifs peuvent être utilisés en particulier à des concentrations allant de 0,0001% à 5% en poids par rapport au poids total de la composition.

L'invention a aussi pour objet un procédé de traitement cosmétique du teint pale, consistant à appliquer sur la peau une composition cosmétique comprenant dans un milieu physiologiquement acceptable, une quantité efficace du manganèse.

L'invention a aussi pour objet un procédé de traitement cosmétique pour combattre la pâleur de la peau, consistant à appliquer sur la peau une composition cosmétique comprenant dans un milieu physiologiquement acceptable, une quantité efficace du manganèse.

Le procédé de traitement cosmétique de l'invention peut être mis en oeuvre notamment en appliquant la composition cosmétique telle que définie ci-dessus, selon la technique d'utilisation habituelle de ces compositions. Par exemple : application de crèmes, de gels, de sérums, de lotions, de laits de démaquillage ou de compositions anti-solaires sur la peau ou de compositions pour spray.

Les exemples et compositions suivants illustrent l'invention. Dans les compositions les proportions indiquées sont des pourcentages en poids, sauf mention contraire.

### Exemple 1 : Activité du manganèse (gluconate de manganèse, chlorure de manganèse et carbonate de manganèse) dans un modèle ex vivo de vaisseaux isolés sans endothélium.

Des anneaux de vaisseaux sanguins sont maintenus dans une cuve de 20 ml remplie de liquide de survie (liquide de Krebs Henseleit) maintenu à une température de 37°C et oxygéné à l'aide d'un mélange d'oxygène 95 % et de CO₂ 5 %.
Les variations de tension du vaisseau sont ensuite enregistrées avec une précharge initiale de plusieurs mg.
Les anneaux sont contractés par la noradrénaline à la concentration de 3.10⁻⁷M. Sur chaque préparation, l'effet des produits à tester a été évalué aux concentrations croissantes et cumulées de 3.10⁻⁸ M à 10⁻⁵ M.

Résultats obtenus dans le modèle de vasoconstriction avec les 3 sels de manganèse.

| Produit | Concentration | % de relaxation |
|---|---|---|
| Gluconate de manganèse (n=6) | 10⁻⁶ M | 100% |
| MnCO₃(n=3) | 10⁻⁶ M | 100% |
| MnCl₂ (n=3) | 10⁻⁶ M | 100% |

Quelle que soit la nature du contre-ion, les sels de manganèse relaxent le tissu contracté qui passe d'un état contracté à un état dilaté.

### Exemple 2 : exemples de compositions selon l'invention.

| Composition 1 : Lotion de soin anti-stress pour le visage | |
|---|---|
| Gluconate de Manganèse | 1,00 % |
| Antioxydant | 0,05 % |
| Conservateur | 0,30 % |
| Ethanol (solvant) | 8,00 % |
| Eau | qsp 100 % |

La lotion obtenue agit sur les rides lors d'une utilisation répétée (application biquotidienne pendant un mois).

| Composition 2 : Gel destressant pour le soin du visage | |
|---|---|
| Gluconate de Manganèse | 1,00 % |
| Hydroxypropylcellulose * | 1,00 % |
| Conservateur | 0,30 % |
| Ethanol (solvant) | 15,00 % |
| Antioxydant | 0,05 % |
| Eau | qsp 100 % |

| | |
|---|---|
| * : Klucel H vendu par la société Hercules (gélifiant). | |

Le gel obtenu agit sur les rides. Il peut être appliqué quotidiennement matin et soir pendant un mois.

| Composition 3 : Crème de soin du visage (émulsion huile-dans-eau) | |
|---|---|
| Carbonate de Manganèse | 0,50 % |
| Stéarate de glycérol (émulsionnant) | 2,00 % |
| Polysorbate 60 (Tween 60 vendu par la société ICI) (émulsionnant) | 1,00 % |
| Acide stéarique | 1,40 % |
| Triéthanolamine (neutralisant) | 0,70 % |
| Carbomer (Carbopol 940 vendu par la société Goodrich) | 0,40 % |
| Fraction liquide de beurre de karité | 12,00 % |
| Perhydrosqualène | 12,00 % |
| Conservateur | 0,30 % |
| Parfum | 0,50 % |
| Antioxydant | 0,05 % |
| Eau | qsp 100 % |

On obtient une crème blanche, onctueuse, qui agit sur les rides et les ridules, et que l'on peut appliquer quotidiennement.

| Composition 4 : Crème de soin détoxifiante du visage (émulsion huile-dans-eau) | |
|---|---|
| Gluconate de Manganèse | 0,50 % |
| Mono-, distéarate de glycérol | 2,00 % |
| Alcool cétylique | 1,50 % |
| Mélange alcool cétylstéarylique/alcool cétylstéarylique oxyéthyléné 33 OE | 7,00 % |
| Diméthylpolysiloxane | 1,50 % |
| Huile de vaseline | 17,50 % |
| Conservateur | 0,30 % |
| Parfum | 0,50 % |
| Glycérine | 12,50 % |
| Eau | qsp 100 % |

## Revendications

1. Utilisation cosmétique d'une quantité efficace du manganèse ou d'au mons l'un de ses sels, dans une composition cosmétique pour relaxer et/ou relâcher le tissu vasculaire cutané et/ou sous-cutané et ainsi combattre la pâleur de la peau.

2. Utilisation d'une quantité efficace du manganèse pour la préparation d'une composition destinée à rétablir l'équilibre vasculaire de la peau modifié après un stress.

3. Utilisation selon l'une quelconque des revendications précédentes, **caractérisée en ce que** le manganèse est utilisé en une quantité représentant de 0,0001% à 10 % du poids total de la composition.

4. Utilisation selon l'une quelconque des revendications précédentes, **caractérisée en ce que** le manganèse est utilisé en une quantité représentant de 0,001 % à 5 % du poids total de la composition.

5. Utilisation selon l'une quelconque des revendications précédentes, **caractérisée en ce que** le manganèse est sous forme ionique ou sous la forme d'un sel ou sous la forme d'un extrait naturel, végétal ou de micro-organismes, riche en manganèse.

6. Utilisation selon l'une quelconque des revendications précédentes, **caractérisée en ce que** le sel de manganèse est choisi parmi les sels organiques ou inorganiques du manganèse.

7. Utilisation selon la revendication 6, **caractérisée en ce que** le sel organique de manganèse est choisi parmi le gluconate de manganèse ou le carbonate de manganèse ou l'acétate de manganèse ou le citrate de manganèse ou l'oléate de manganèse ou l'oxalate de manganèse.

8. Utilisation selon la revendication 6, **caractérisée en ce que** le sel inorganique de manganèse est choisi parmi les sels minéraux comme le chlorure de manganèse ou le borate de manganèse ou le nitrate de manganèse ou le phosphate de manganèse ou le sulfate de manganèse.

9. Procédé de traitement cosmétique de la pâleur de la peau, consistant à appliquer par voie topique une composition cosmétique comprenant dans un milieu physiologiquement acceptable une quantité efficace du manganèse.

## Patentansprüche

1. Kosmetische Verwendung einer wirksamen Menge von Mangan oder mindestens eines Mangansalzes in einer kosmetischen Zusammensetzung zur Entspannung und/oder Lockerung des kutanen und/ oder subkutanen vaskulären Gewebes und somit zur Bekämpfung der Blässe der Haut.

2. Verwendung einer wirksamen Menge von Mangan zur Herstellung einer Zusammensetzung, die dazu vorgesehen ist, das nach einer Stress-Situation veränderte vaskuläre Gleichgewicht der Haut wiederherzustellen.

3. Verwendung nach einem der vorhergehenden Ansprüche, **dadurch gekennzeichnet, dass** das Mangan in einer Menge von 0,0001 bis 10 % des Gesamtgewichts der Zusammensetzung verwendet wird.

4. Verwendung nach einem der vorhergehenden Ansprüche, **dadurch gekennzeichnet, dass** das Mangan in einer Menge von 0,001 bis 5 % des Gesamtgewichts der Zusammensetzung verwendet wird.

5. Verwendung nach einem der vorhergehenden Ansprüche, **dadurch gekennzeichnet, dass** das Mangan in ionischer Form oder in Form eines Salzes oder in Form eines Naturextraktes, Pflanzenextraktes oder Extraktes von Mikroorganismen, der einen hohen Mangangehalt aufweist, vorliegt.

6. Verwendung nach einem der vorhergehenden Ansprüche, **dadurch gekennzeichnet, dass** das Mangansalz unter den organischen oder anorganischen Mangansalzen ausgewählt ist.

7. Verwendung nach Anspruch 6, **dadurch gekennzeichnet, dass** das organische Mangansalz unter Mangangluconat, Mangancarbonat, Manganacetat, Mangancitrat, Manganoleat oder Manganoxalat ausgewählt ist.

8. Verwendung nach Anspruch 6, **dadurch gekennzeichnet, dass** das anorganische Mangansalz unter den Mineralsalzen wie Manganchlorid, Manganborat, Mangannitrat, Manganphosphat oder Mangansulfat ausgewählt ist.

9. Verfahren zur kosmetischen Behandlung der Blässe der Haut, das darin besteht, auf topischem Wege eine Zusammensetzung aufzubringen, die in einem physiologisch akzeptablen Medium eine wirksame Menge von Mangan enthält.

## Claims

1. Cosmetic use of an effective amount of manganese or of at least one of its salts, in a cosmetic composition, to relax and/or slacken cutaneous and/or subcutaneous vascular tissue and thus to combat skin pallor. ,

2. Use of an effective amount of manganese for the preparation of a composition intended to re-establish the skin's vascular equilibrium which has been modified after a stress episode.

3. Use according to either of the preceding claims, **characterized in that** the manganese is used in an amount representing from 0.0001% to 10% of the total weight of the composition.

4. Use according to any one of the preceding claims, **characterized in that** the manganese is used in an amount representing from 0.001% to 5% of the total weight of the composition.

5. Use according to any one of the preceding claims, **characterized in that** the manganese is in ionic form, in the form of a salt or in the form of a manganese-rich natural, plant or microorganism extract.

6. Use according to any one of the preceding claims, **characterized in that** the manganese salt is chosen from organic and inorganic manganese salts.

7. Use according to Claim 6, **characterized in that** the organic manganese salt is chosen from manganese gluconate, manganese carbonate, manganese acetate, manganese citrate, manganese oleate and manganese oxalate.

8. Use according to Claim 6, **characterized in that** the inorganic manganese salt is chosen from mineral salts, for instance manganese chloride, manganese borate, manganese nitrate, manganese phosphate or manganese sulphate.

9. Cosmetic treatment process for skin pallor, which consists in topically applying a cosmetic composition comprising an effective amount of manganese in a physiologically acceptable medium.
